# EUROPEAN PATENT SPECIFICATION

(11) **EP 1 834 628 B1**
(45) Date of publication and mention of the grant of the patent: **29.12.2010**
(21) Application number: 07425117.4
(22) Date of filing: 01.03.2007
(51) Int. Cl.: A61K 8/37, A61K 8/49, A61K 8/63, A61K 31/231, A61K 31/352, A61K 31/575, A61Q 7/02, A61P 43/00, A61K 8/97

(54) **Cosmetic composition comprising phytosterols, isoflavones and methylricinoleate, use as inhibitor of 5-alpha-reductase and hair growth**
Kosmetische Zusammensetzung enthaltend Phytosterole, Isoflavone und Ricinolsäuremethylester, Verwendung zur Hemmung der 5-Alpha-reductase und des Haarwuchses
Composition cosmétique contenant des phytostérols, des isoflavones et du ricinoléate de méthyl, Utilisation comme inhibiteur de la 5-alpha-réductase et de la pousse du cheveu

(30) Priority: 03.03.2006 IT MI20060392
(43) Date of publication of application: 19.09.2007
(73) Proprietor: The First S.p.A., 20126 Milano (IT)
(72) Inventor: Bocchietto, Elena, 28046 Meina (Novara) (IT)
(74) Representative: Long, Giorgio

(56) References cited:
- WO-A-01/52837
- FR-A1- 2 791 255
- US-A1- 2005 003 024
- US-A1- 2005 147 631

## Description

The present invention relates to a cosmetic composition used to inhibit hair growth.

In both men and women, hair growth is stimulated by androgens present in skin cells. In particular, at the cellular level, testosterone is converted into 17-beta-hydroxy-5-alpha-androstan-3-one or 5-dihydroxytestosterone (DHT) by the enzyme 5-alpha-reductase. DHT has higher hair growth stimulating activity to that of testosterone. Indeed, unlike testosterone, it cannot be converted to oestrogens, thus exercising exclusively androgenic activity and, furthermore, has a higher affinity than testosterone for the androgen receptor.

The enzyme 5-alpha-reductase is present in two isoforms, wherein one isoform, 5-alpha-Rl, is predominantly expressed in the skin and prostate. 5-alpha-reductase R1 is also found in melanocytes, in microcircle endothelial cells, in fibroblasts and in hair dermal papillae, which represent all the cell types targeted by androgens. The R2 isoform is also found in skin, although in somewhat lesser amounts.

Hence, the 5-alpha-reductases plays an essential role in the regulation of hair growth.

Molecules which inhibit 5-alpha-reductase have an anti-androgenic effect, and are hence thus useful in counteracting the growth of body hair.

The problem addressed by the present invention is that of providing a cosmetic composition for topical use, capable of inhibiting hair growth.

Said problem is resolved by a cosmetic composition as defined in the appended claims.

The present invention relates to a cosmetic composition comprising phytosterols in quantities ranging from 1 to 20% by weight, isoflavones from 0.1 to 4% by weight and methylricinoleate from 80 to 90% by weight. Preferably, the composition of the invention comprises phytosterols in quantities ranging from 12 to 17% by weight, isoflavones from 1 to 2% by weight and methylricinoleate in quantities ranging from 82 to 85% by weight. Phytosterols and isoflavones are well known naturally-derived products which can be obtained synthetically or extracted from plants, such as soya, maize, sesame or other cereal grains. In particular, phytosterols are mainly found in the non-saponifiable fractions of vegetable oils and fats, with soya representing the most important commercial source.

They have structural formulae analogous to that of human sterols and are known inhibitors of the enzyme 5-alpha-reductase, thus impeding the conversion of testosterone to DHT. They also possess marked anti-inflammatory properties, acting on the arachidonic acid cascade and reducing leukotriene levels.

Phytosterols are easily absorbed through the skin and act on skin cells at numerous levels.

Soya isoflavones have antioxidant and dermoprotective properties, which contribute towards enhancing the anti-inflammatory role of phytosterols.

In the present cosmetic composition the use of extracted phytosterols and isoflavones is preferred.

Preferably, the composition comprises between 1 and 20% by weight of a plant phytosterol extract, between 0.1 and 4% of a plant isoflavone extract, and between 80 and 90% of methylricinoleate.

Advantageously, the composition of the invention comprises between 11 and 17% by weight of a plant phytosterol extract, between 1 and 2% of a plant isoflavone extract, and between 80 and 90% of methylricinoleate.

In particular, the plant phytosterol extract contains from 40 to 60% by weight sitosterol, from 18 to 30% by weight campesterol and from 15 to 28% by weight stigmasterol. The plant isoflavone extract contains approx 100% isoflavones. Preferably, the plant phytosterol and isoflavone extracts are soya, maize, sesame or other cereal grain extracts. More preferably, said plant extracts are soya bean extracts. Methylricinoleate is a synthetic product, having an oily form, and which has 5-alpha-reductase inhibitory properties.

It has surprisingly been found that by mixing the phytosterols, isoflavones and methylricinoleate in the above described quantities, a cosmetic composition is obtained with hair growth inhibitory activity which is unexpectedly greater with respect to the activity that might have been reasonably expected by summing the activities of the individual components. In other words, the cosmetic composition of the invention has synergistic activity that is thus greater than the sum of the activities of the individual components.

The cosmetic composition of the invention is formulated using excipients normally used in the cosmetic sector. The excipients used in the cosmetic formulation of the present invention are selected from the group consisting of: solvents, emollients, hygroscopic agents, hydrating agents, filmogenic agents, preservatives, surfactants, viscosity regulators, antioxidants, emulsifiers, fragrances, buffers, chelating agents, conditioners and mixtures thereof. More particularly, the aforementioned cosmetic excipients (indicated by their INCI-EU names) are selected from the group consisting of: PPG-15 Stearyl ether, isohexadecane, dimethicone, ethylhexyl ethylhexanoate, cocoglycerides, lecithin, propylene glycol, glycerin, hexyldecanol, betaine, glyceryl polymethacrylate, acrylates/C10-30 alkyl acrylate crosspolymer, polyacrylamide, polymethyl methacrylate, phenoxyethanol, methylparaben, butylparaben, ethylparaben, propylparaben and isobutylparaben, carbomer, disodium EDTA, aminomethyl propanol, citric acid, perfume, cetearyl alcohol, cetearyl glucoside, potassium cetyl phosphate, laureth-7, water, C13-C14 isoparaffin, ceramide 3, tocopherol, ascorbyl palmitate, ethyl xymeninate and mixtures thereof.

When present in the cosmetic formulation, the aforementioned excipients are included in the quantities indicated in table 1.

**Table 1**

| **Excipient (Chemical or INCI-EU name)** | **Role** | **Quantity (% by weight)** | **Preferred quantity (% by weight)** |
|---|---|---|---|
| WATER | Solvent | to 100 | to 100 |
| PPG-15 STEARYL ETHER | Emollient | 2-9 | 5-9 |
| ISOHEXADECANE | Emollient | 2-9 | 5-9 |
| PROPYLENE GLYCOL | Hygroscopic agent | 0.1-6 | 2-5 |
| BETAINE | Hydrating agent | 0.1-3 | 0.5-2.0 |
| ETHYL XYMENINATE | conditioner | 0.01-1 | 0.02-0.5 |
| GLYCERYL POLYMETHACRYLATE | Filmogenic agent | 0.5-2 | 0.5-1 |
| DIMETHICONE | Emollient | 0,1-3 | 1-2 |
| PHENOXYETHANOL | Preservative | 0.1-0.9 | 0.6-0.8 |
| POTASSIUM CETYL PHOSPHATE | surfactant | 0.1-2 | 0.1-0.6 |
| CARBOMER | Viscosity regulator | 0.1-2 | 0.5-1.0 |
| FRAGRANCE | Fragrance | 0.1-1.0 | 0.1-0.3 |
| AMINOMETHYL PROPANOL | Buffer | 0.1-1 | 0.1-0.5 |
| ACRYLATES/C10-30 ALKYL ACRYLATE CROSSPOLYMER | Filmogenic agent | 0.1-3 | 0.1-1 |
| METHYLPARABEN | Preservative | 0.05-0.2 | 0.1-0.2 |
| DISODIUM EDTA | cheating agent | 0.05-0.1 | 0.1. |
| BUTYLPARABEN | Preservative | 0.01-0.2 | 0.1-0.2 |
| ETHYLPARABEN | Preservative | 0.01-0.2 | 0.01-0.05 |
| PROPYLPARABEN | Preservative | 0.01-0.2 | 0.01-0.05 |
| ISOBITTYLPARABEN | Preservative | 0.01-0.2 | 0.01-0.05 |
| ETHYLHEXYL ETHYLHEXANOATE | Emollient | 4-12 | 5-9 |
| COCOGLYCERIDES | EmolUent/Emulsifier | 4-12 | 5-9 |
| GLYCERIN | Denammt/Hygroseopic agent/ Solvent | 1-5 | 2-3 |
| HEXYLDECANOL | Hygroscopic agent | 0.5-3 | 0.5-1 |
| POLYACRYLAMIDE | Antistatic agent/Binding agent/Filmogenic agent | 0.2-2 | 0.5-1 |
| C13-14 ISOPARAFFIN | Solvent | 0.01 - 0.2 | 0.01-0.5 |
| LAURETH-7 | Emulsifier/Surfactant | 0.01 - 0.2 | 0.01-0.5 |
| LECITHINE | Conditioner/Emollient/ Emulsifier | 0.01 - 0.2 | 0.01-0.5 |
| CERAMIDE 3 | Conditioner | 0.01 - 1.0 | 0.01-0.5 |
| TOCOPHEROL | Antioxidant | 0.001-1.0 | 0.05-0.5 |
| ASCORBYL PALMITATE | Antioxidant | 0.001-1.0 | 0.05-0.5 |
| CITRIC ACID | Buffer/ Chelating agent | 0.001-0.1. | 0.05-0.1 |
| DIMETHICONE, POLYMETHYL METHACRYLATE POLYACRYLAMIDE | Polymers, Filmogenic agent and silicones | 0.5-5.0. | 0.5-2 |

The active cosmetic composition of the invention comprising phytosterols, isoflavones and methylricinoleate is present in the cosmetic formulation in quantities ranging from 1 to 30%, preferably from 2 to 20% by weight. Hereinafter, this composition is referred to as "BIOCOMPLEX".

The cosmetic formulation of the invention can be in the form of a cream, gel, emulsion or lotion, preferably as an emulsion.

The aforementioned cosmetic formulations are prepared in conventional ways, well known in the sector.

Examples of preferred formulations of the cosmetic composition of the invention (BIOCOMPLEX) are reported in the following tables 2-4.

**Table 2**

| **Chemical or INCI-EU name** | **Role** | **Quantity** **%** | **stage** |
|---|---|---|---|
| WATER | Solvent | to 100 | A |
| PPG-15 STEARYL ETHER | Emollient | 2-9 | B |
| ISOHEXADECANE | Emollient | 2-9 | B |
| PROPYLENE GLYCOL | Hygroscopic agent | 0.1-6 | A |
| BETAINE | Hydrating agent | 0.1-3 | A |
| ETHYL XYMENINATE | conditioner | 0.01-1, | D |
| GLYCERYLPOLYMETHACRYLATE | ilmogenic agent | 0.5-2 | B |
| DIMETHICONE | Emollient | 0.1-3 | B |
| BIO COMPLEX | active principle | 4-10 | C |
| PHENOXYETHANOL | Preservative | 0.1-0.9 | E |
| POTASSIUM CETYL PHOSPHATE | surfactant | 0.1-2 | B |
| CARBOMER | Viscosity regulator | 0.1-2 | A |
| FRAGRANCE | Fragrance | as required. | G |
| AMINOMETHYLPROPANOL | Buffer | 0.1-1 | F |
| ACRYLATES/C10-30 ALKYL ACRYLATE CROSSPOLYMER | Filmogenic agent | 0.1-3 | H |
| METHYLPARABEN | Preservative | 0.05-0.2 | E |
| DISODIUM EDTA | Chelating agent | 0.05-0.1 | A |
| BUTYLPARABEN | Preservative | 0.01-0.2 | E |
| ETHYLPARABEN | Preservative | 0.01 | E |
| PROPYLPARABEN | Preservative | 0.01 | E |
| ISOBUTYLPARABEN | Preservative | 0.01 | E |

In table 2, BIOCOMPLEX comprises phytosterols, isoflavones and methylricinoleate, in quantities ranging from 13 to 17%, 1 to 2% and 80 to 88% respectively.

The cosmetic formulation reported in table 2 is an emulsion obtained by means of the following process.

The stage A components are dissolved in the formula quantity of water in an appropriate mixer and heated to 65-70°C with constant mixing.

The stage B components are mixed to homogeneity in a suitable melting device and heated to 65-70°C, and then the stage C components added to those of stage B. Afterwards, the stage B components added to those of stage A, homogenising the mixture with a turbine mixer under vacuum followed by mixing using a blade and counter blade mixer.

To stage B+A are added the stage E components, previously partially solubilised, and then the stage H components, with thorough mixing.

Finally, when the temperature has dropped to below 50°C, the stage F components, and then the stages D and G components are added, mixing to homogeneity using a blade and counter blade mixer.

**Table 3**

| **Chemical or INCI-EU name** | **Role** | **Quantity** **%** | **STAGE** |
|---|---|---|---|
| WATER | Solvent | to 100 | A |
| ETHYLHEXYL ETHYLHEXANOATE | Emollient | 4-12 | B |
| COCOGLYCERIDES | Emollient/Emulsifier | 4-12 | B |
| GLYCERIN | Denaturant/Hygroscopic agent/ Solvent | 1-5 | A |
| DIMETHICONE | Antifoaming agent/Emollient | 0.5-5 | B |
| BIO COMPLEX | Active principle | 1-10 | C |
| HEXYLDECANOL | Hygroscopic agent | 0,5-3 | B |
| PHENOXYETHANOL | Preservative | 0.1-0.9 | D |
| FRAGRANCE | Deodorant | as required | E |
| POTASSIUM CETYL PHOSPHATE | surfactant | 0.500 | B |
| AMINOMETHYL PROPANOL | Buffer | 0.1-2 | F |
| CARBOMER | Viscosity regulator | 0.1-2 | A |
| ACRYLATES/C10-30 ALKYL ACRYLATE CROSSPOLYMER | Filmogenic agent | 0.1-2 | G |
| POLYACRYLAMIDE | Antistatic agent/Binding agent/Filmogenic agent | 0.21-2 | |
| METHYLPARABEN | Preservative | 0.01-0.2 | |
| C13-14 ISOPARAFFIN | solvent | 0.01-0.2 | A |
| DISODIUM EDTA | Chelating agent/Viscosity regulator | 0.01 - 0.2 | |
| LAURETH-7 | Emulsifier/Surfactant | 0.01 - 0.2 | A |
| BUTYLPARABEN | Preservative | 0.01 - 0.2 | D |
| METHYLPARABEN | Preservative | 0.01 - 0.2 | D |
| LECITHIN | Conditioner/Emollient/ Emulsifier | 0.01 - 0.2, | H |
| CERAMIDE 3 | Conditioner | 0.01 - 0.2 | I |
| PROPYLPARABEN | Preservative | 0.01 - 0.2 | D |
| ISOBUTYLPARABEN | Preservative | 0.01 - 0.2 | D |
| TOCOPHEROL | Antioxidant | 0.001-0.1 | H |
| ASCORBYL PALMITATE | Antioxidant | 0.001-0.1 | H |
| CITRIC ACID | Buffer/ Chelating agent | 0.001-0.1 | H |

In table 3, BIOCOMPLEX comprises phytosterols, isoflavones and methylricinoleate, in quantities ranging from 13 to 17%, 1 to 2% and 80 to 88% respectively. The cosmetic formulation reported in table 3 is an emulsion obtained by means of the following process.

The stage A components are dissolved in the formula quantity of water in an appropriate mixer and heated to 65-70°C with constant mixing.

The stage B components are mixed to homogeneity in a suitable melting device and heated to 65-70°C, and then the stage C components added to those of stage B. Afterwards, the stage B components added to those of stage A, homogenising the mixture with a turbine mixer under vacuum followed by mixing using a blade and counter blade mixer.

To stage B+A are added the components of stage D, previously partially solubilised, then the stage G components, while mixing thoroughly.

Finally, when the temperature was below 50°C, the stage F and then stage E components added, followed by the premixed stage G components, and the entire preparation mixed to homogeneity using a blade and counter blade mixer.

A further preferred example cosmetic formulation according to the invention is reported in table 4.

**Table 4**

| **CAS N°** | **Chemical or INCI-EU name** | **Role** | **Quantity %** | **stage** |
|---|---|---|---|---|
| 7732-18-5 | WATER | Solvent | to 100 | A |
| 25231-21-04 | PPG-15 STEARYL ETHER | Emollient | 7.00 | B |
| 4390-04-9 | ISOHEXADECANE | Emollient | 6.000 | B |
| 67762-27-0 | CETEARYL ALCOHOL | Emulsifier | 4.000 | B |
| 56-81-5 | GLYCERIN | Hygroscopic agent | 2.000 | A |
| 246159-33-1 | CETEARYL GLUCOSIDE | Emulsifier | 1.000 | B |
| 9006-65-9 | DIMETHICONE | Antifoaming agent | 1.000 | B |
| 122-99-6 | PHENOXYETHANOL | Preservative | 0.700 | A |
| | BIO COMPLEX | active principle | 6.000 | C |
| 84861-79-0 | POTASSIUM CET-YL PHOSPHATE | surfactant | 0.500 | B |
| | FRAGRANCE | fragrance | 0.500 | D |
| 70445-33-9 | ETHYLHEXYL GLYCERIN | Hygroscopic agent | 0.500 | A |
| 9003-05-8 | POLYACRYLAMIDE | Texturing factor | 0.200 | A |
| 99-76-3 | METHYLPARABEN | Preservative | 0.160 | E |
| 246538-79-4 | C13-14 ISOPARAFFIN | Solvent | 0.120 | A |
| 139-33-3 | DISODIUM EDTA | Chelating agent | 0.1000 | A |
| 3055-97-8 | LAURETH-7 | Emulsifier/Surfactant | 0.040 | A |
| 94-26-8 | BUTYLPARABEN | Preservative | 0.040 | E |
| 120-47-8 | ETHYLPARABEN | Preservative | 0.040 | E |
| 94-13-3 | PROPYLPARABEN | Preservative | 0.020 | E |
| 4247-02-3 | ISOBUTYLPARABEN | Preservative | 0.020 | E |

By the term BIOCOMPLEX is meant a mixture comprising phytosterols, isoflavones and methylricinoleate, in quantities of 15%, 1% and 84% respectively.

The aforementioned emulsion is prepared in the following manner. The stage A components are dissolved in the formula quantity of water in an appropriate mixer and heated to 65-70°C.

The stage B components are mixed to homogeneity in a suitable melting device and heated to 65-70°C, and then the stage C components added to those of stage B. Afterwards, the stages C+B components are added to those of stage A, homogenising by means of a turbine mixer under vacuum, and then mixing using a blade and counter blade mixer, and then the previously solubilised stage E components added.

When the temperature is below 50°C, the stage D components are added and then the preparation mixed to homogeneity using a blade and counter blade mixer.

### ACTIVITY TESTS

### In vitro testing

The *in vitro* ability of a cosmetic composition of the invention for topical use to modulate 5-alpha-reductase activity in fibroblasts has been assessed.

The aim of this test is to quantitatively establish the effects of the product under test on 5α-reductase activity in a murine fibroblast cell line.

In the test in question, fibroblasts are treated with testosterone in order to increase basal production of 5a-reductase, and then with the product under test at several concentrations for various periods of time up to 72 hours. At various endpoints (in this specific case at 48 and 72 hours) the DHT titre produced by the treated cells is measured by means of an ELISA method, and by comparison with untreated cells, the specific percentage inhibition of enzyme activity can be estimated.

A titrated extract of *Serenoa repens* (Saw palmetto), a plant well known in the pharmacopoeia for its 5a-reductase inhibitory capacity, and used for that purpose in the treatment of prostatic hyperplasia in men, is used as a positive control.

The samples tested *in vitro* are:
A) The cosmetic composition of the invention (BIOCOMPLEX) comprising 17% phytosterols, 1% isoflavones and 82% methylricinoleate;
B) Phytosterol titred soya extract, comprising 17% phytosterols and 83% Vaseline oil.

The murine cells have been seeded in the presence of testosterone on 24 well plates in DMEM culture medium + 10% FCS containing the product under test at the final concentrations indicated in table 5 below, with 10µg/ml *Serenoa repens* used as a positive control. Exposure has been continued up to 72 hours with 5% CO₂, considering 2 endpoints (48 and 72 hours). The culture medium has been replaced daily.

Each sample has been assayed in triplicate. Cells treated with testosterone alone have been used as a negative control. At the two endpoints, cytotoxicity tests (MTT) and ELISA assays have been performed to determine the quantities of DHT present. 50 µL of culture medium has been withdrawn from each well, to be used to quantify DHT by means of an ELISA assay, performed at room temperature at each end-point (48 and 72 hours).

The method envisages the preparation of a standard curve, calculated within a suitable concentration interval.

The ELISA assay uses a specific antibody against human DHT immobilised on a solid substrate, and a reagent (DHT conjugated to Horse-Radish-Peroxidase) which competes for antibody binding.

The reaction is detected by means of a substrate solution which develops a coloured compound, the intensity of which is inversely proportional to the concentration of DHT present.

The absorbance at 450 nm is read for each sample.

The results obtained, expressed as percentage inhibition of 5-α-reductase, are reported in table 5.

**Table 5**

| | **48 hours** | | **72 hours** | |
|---|---|---|---|---|
| | **DHT (pg/ml)** | **% inhibition** | **DHT (pg/ml)** | **% inhibition** |
| **Negative control** | 1495.0 | | 3694.4 | |
| **Sample A (1 mg/ml)** | 873.9 | 41.5 | 420.6 | 88.6 |
| **Sample B (1 mg/ml)** | 1027.3 | 31.3 | 886.4 | 76.0 |
| **10 µg/ml *Serenoa repens* (positive control)** | 438.9 | 70.6 | 320.6 | 91.3 |

The results show that, when tested at equal concentrations (1 mg/ml) and phytosterol titre, product A is more efficacious than product B, and is comparable to the *Serenoa repens* used as positive control.

The results also show a synergistic effect of the cosmetic composition of the invention. In practice, mixing phytosterols and methylricinoleate, with known 5-alpha-reductase inhibitory activities, with isoflavones, having antioxidant properties, results in an inhibitory capacity greater than that which might have been expected by simply summing the activities of the individual components.

## Claims

1. A cosmetic composition comprising phytosterols in quantities ranging from 1 to 20% by weight, isoflavones from 0.1 to 4% by weight and methylricinoleate from 80 to 90% by weight.

2. The composition according to claim 1 wherein said phytosterols are present in quantities ranging from 12 to 17% by weight, said isoflavones are present in quantities ranging from 1 to 2% by weight, and said methylricinoleate is present in quantities ranging from 82 to 85% by weight.

3. The composition according to claims 1 or 2 wherein said phytosterols are a plant phytosterol extract comprising from 40 to 60% by weight sitosterol, from 18 to 30% by weight campesterol and from 15 to 28% by weight stigmasterol.

4. The composition according to any of the claims 1 to 3 wherein said isoflavones are a plant isoflavone extract comprising approx. 100% isoflavones by weight.

5. The composition according to claims 3 or 4 wherein said plant phytosterol and isoflavone extracts are soya bean extracts.

6. A cosmetic formulation comprising an active cosmetic composition according to any of the claims 1 to 5 along with cosmetically acceptable excipients.

7. The formulation according to claim 6 comprising the active cosmetic composition according to any of the claims 1 to 5 in quantities ranging from 1 to 30% by weight, preferably from 2 to 20% by weight.

8. The formulation according to claims 6 or 7 wherein said excipients are selected from the group consisting of: solvents, emollients, hygroscopic agents, hydrating agents, filmogenic agents, preservatives, surfactants, viscosity regulators, antioxidants, emulsifiers, fragrances, buffers, chelating agents, conditioners and mixtures thereof.

9. The formulation according to any of the claims 6 to 8 wherein said excipients are selected from the group consisting of: PPG-15 Stearyl ether, isohexadecane, dimethicone, ethylhexyl ethylhexanoate, cocoglycerides, lecithin, propylene glycol, glycerin, hexyldecanol, betaine, glyceryl polymethacrylate, acrylates/C10-30 alkyl acrylate crosspolymer, polyacrylamide, polymethyl methacrylate, phenoxyethanol, methylparaben, butylparaben, ethylparaben, propylparaben and isobutylparaben, carbomer, disodium EDTA, aminomethyl propanol, citric acid, perfume, cetearyl alcohol, cetearyl glucoside, potassium cetyl phosphate, laureth-7, water, C13-C14 Isoparaffin, ceramide 3, tocopherol, ascorbyl palmitate, ethyl xymeninate and mixtures thereof.

10. The formulation according to claim 9 wherein said excipients are present in the quantities reported in the following table:
| **Excipient (Chemical or INCI-EU name)** | **Role** | **Quantity (% by weight)** | **Preferred quantity (% by weight)** |
|---|---|---|---|
| WATER | Solvent | to 100 | to 100 |
| PPG-15 STEARYL ETHER | Emollient | 2-9 | 5-9 |
| ISOHEXADECANE | Emollient | 2-9 | 5-9 |
| PROPYLENE GLYCOL | Hygroscopic agent | 0.1-6 | 2-5 |
| BETAINE | Hydrating agent | 0.1-3 | 0.5-2.0 |
| ETHYL XYMENINATE | conditioner | 0.01-1 | 0.02-0.5 |
| GLYCERYL POLYMETHACRYLATE | Filmogenic agent | 0.5-2 | 0.5-1 |
| DIMETHICONE | Emollient | 0.1-3 | 1-2 |
| PHENOXYETHANOL | Preservative | 0.1-0.9 | 0.6-0.8 |
| POTASSIUM CETYL PHOSPHATE | surfactant | 0.1-2 | 0.1-0.6 |
| CARBOMER | Viscosity regulator | 0.1-2 | 0.5-1.0 |
| FRAGRANCE | Fragrance | 0.1-1.0 | 0.1-0.3 |
| AMINOMETHYL PROPANOL | Buffer | 0.1-1 | 0.1-0.5 |
| ACRYLATES/C10-30 ALKYL ACRYLATE CROSSPOLYMER | Filmogenic agent | 0.1-3 | 0.1-1 |
| METHYLPARABEN | Preservative | 0.05-0.2 | 0.1-0.2 |
| DISODIUM EDTA | Chelating agent | 0.05-0.1 | 0.1 |
| BUTYLPARABEN | Preservative | 0.01-0.2 | 0.1-0.2 |
| ETHYLPARABEN | Preservative | 0.01-0.2 | 0.01-0.05 |
| PROPYLPARABEN | Preservative | 0.01-0.2 | 0.01-0.05 |
| ISOBUTYLPARABEN | Preservative | 0.01-0.2 | 0.01-0.05 |
| ETHYLHEXYL ETHYLHEXANOATE | Emollient | 4-12 | 5-9 |
| COCOGLYCERIDES | Emollient/Emulsifier | 4-12 | 5-9 |
| GLYCERIN | Denaturant/Hygroscopic agent/ Solvent | 1-5 | 2-3 |
| HEXYLDECANOL | Hygroscopic agent | 0.5-3 | 0.5-1 |
| POLYACRYLAMIDE | Antistatic agent/Binding agent/Filmogenic agent | 0.2-2 | 0.5-1 |
| G13-14ISOPARAFFIN | Solvent | 0.01-0.2 | 0.01-0.5 |
| LAURETH-7 | Emulsifier/Surfactant | 0.01-0.2 | 0.01-0.5 |
| LECITHINE | Conditioner/Emollient/ Emulsifier | 0.01-0.2 | 0.01-0.5 |
| CERAMIDE 3 | Conditioner | 0.01-1.0 | 0.01-0.5 |
| TOCOPHEROL | Antioxidant | 0.001-1.0 | 0.05-0.5 |
| ASCORBYL PALMITATE | Antioxidant | 0.001-1.0 | 0,05-0.5 |
| CITRIC ACID | Buffer/ Chelating agent | 0.001-0.1 | 0.05-0.1 |
| DIMETHICONE, POLYMETHYL METHACRYLATE POLYACRYLAMIDE | Polymers, Filmogenicagent and silicones | 0.5-5.0 | 0.5-2 |

11. The cosmetic formulation according to any of the claims 6 to 10 having the composition shown in the following table:
| **Chemical or INCI-EU name** | **Role** | **Quantity %** |
|---|---|---|
| WATER | Solvent | to 100 |
| PPG-15 STEARYL ETHER | Emollient | 2-9 |
| ISOHEXADECANE | Emollient | 2-9 |
| PROPYLENE GLYCOL | Hygroscopic agent | 0.1-6 |
| BETAINE | Hydrating agent | 0.1-3 |
| ETHYL XYMENINATE | conditioner | 0.01-1 |
| GLYCERYL POLYMETHACRYLATE | Filmogenic agent | 0.5-2 |
| DIMETHICONE | Emollient | 0.1-3 |
| Cosmetic composition comprising: from 13 to 17% by weight phytosterols, from 1 to 2% by weight isoflavones and from 80 to 88% by weight methylricinoteate. | active principle | 4-10 |
| PHENOXYETHANOL | Preservative | 0.1/0.9 |
| POTASSIUM CETYL PHOSPHATE | surfactant | 0.1-2 |
| CARBOMER | Viscosity regulator | 0.1-2 |
| FRAGRANCE | Fragrance | as required |
| AMINOMETHYL PROPANOL | Buffer | 0.1-1 |
| ACRYLATES/C10-30 ALKYL ACRYLATE CROSSPOLYMER | Fllmogenic agent | 0.1-3 |
| METHYLPARABEN | Preservative | 0.05-0.2 |
| DISODIUM EDTA | Chelating agent | 0.05-0.1 |
| BUFYLPARABEN | Preservative | 0.01-0.2 |
| ETHYLPARABEN | Preservative | 0.01 |
| PROPYLPARABEN | Preservative | 0.01 |
| ISOBUTYLPARABEN | Preservative | 0.01 |

12. The cosmetic formulation according to any of the claims 6 to 10 having the composition shown in the following table:
| **Chemical or INCI-EU name** | **Role** | **Quantity %** |
|---|---|---|
| WATER | Solvent | to 100 |
| ETHYLHEXYL ETHYLHEXANOATE | Emollient | 4-12 |
| COCOGLYCERIDES | Emollient/Emulsifier | 4-12 |
| GLYCERIN | Denaturent/Hygroscopic agent/ Solvent | 1-5 |
| DIMETHICONE | Antifoaming agent/Emottient | 0.5-5 |
| Cosmetic composition comprising: from 13 to 17% by weight phytosterols, from 1 to 2% by weight isoflavones and from 80 | Active principle | 1-10 |
| to 88% by weight methylricinoleate. | | |
| HEXYLDECANOL | Hygroscopic agent | 0.5-3 |
| PHENOXYETHANOL | Preservative | 0.1-0.9 |
| FRAGRANCE | Deodorant | as required |
| POTASSIUM CETYL PHOSPHATE | surfactant | 0.500 |
| AMINOMETHYL PROPANOL | Buffer | 0.1-2 |
| CARBOMER | Viscosity regulator | 0.1-2 |
| ACRYLATES/C10-30 ALKYL ACRYLATE CROSSPOLYMER | Filmogenic agent | 0.1-2 |
| POLYACRYLAMIDE | Antistatic agent/Binding agent/Filmogenic agent | 0.21-2 |
| METHYLPARABEN | Preservative | 0.01-0.2 |
| C13-14 ISOPARAFFIN | Solvent | 0.01- 02 |
| DISODIUM EDTA | Chelating agent/Viscosity regulator | 0.01- 0.2 |
| LAURETH-7 | Emulsifier/Surfactant | 0.01 - 0.2 |
| BUTYLPARABEN | Preservative | 0.01 - 0.2 |
| ETHYLPARABEN | Preservative | 0.01-0.2 |
| LECITHIN | Conditioner/Emollient/ Emulsifier | 0.01- 0.2 |
| CERAMIDE 3 | Conditioner | 0.01-0.2 |
| PROPYLPARABEN | Preservative | 0.01-0.2 |
| ISOBUTYLPARABEN | Preservative | 0.01-0.2 |
| TOCOPHEROL | Antioxidant | 0.001-0.1 |
| ASCORBYL PALMITATE | Antioxidant | 0.001-0.1 |
| CITRIC ACID | BufFer/ Chelating agent | 0.001-0.1 |

13. The cosmetic formulation according to any of the claims 6 to 12 having the composition shown in the following table:
| **Chemical or INCI-EU name** | **Role** | **Quantity %** |
|---|---|---|
| WATER | Solvent | to 100 |
| PPG-15 STEARYL ETHER | Emollient | 7.00 |
| ISOHEXADECANE | Emollient | 6.000 |
| CETEARYL ALCOHOL | Emulsifier | 4.000 |
| GLYCERIN | Hygroscopic agent | 2.000 |
| CETEARYL GLUCOSIDE | Emulsifier | 1.000 |
| DIMETHICONE | Antifoaming agent | 1.000 |
| PHENOXYETHANOL | Preservative | 0.700 |
| Cosmetic composition comprising: 15% phytosterols, 1% isoflavones and 84% methylrianoleate | active principle | 6.000 |
| POTASSIUM CETYL PHOSPHATE | surfactant | 0.500 |
| FRAGRANCE | fragrance | 0.500 |
| ETHYLHEXYL GLYCERIN | Hygroscopic agent | 0.500 |
| POLYACRYLAMIDE | Texturing factor | 0.200 |
| METHYLPARABEN | Preservative | 0.160 |
| | | |
|---|---|---|
| C13-14 ISOPARAFFIN | Solvent | 0.120 |
| DISODIUM EDTA | Chelating agent | 0.1000 |
| LAURETH-7 | Emulsifier/Surfactant | 0.040 |
| BUTYLPARABEN | Preservative | 0.040 |
| ETHYLPARABEN | Preservative | 0.040 |
| PROPYLPARABEN | Preservative | 0.020 |
| ISOBUTYLPARABEN | Preservative | 0.020 |

## Patentansprüche

1. Kosmetische Zusammensetzung umfassend Phytosterole in Mengen im Bereich von 1 bis 20 Gew.-%, Isoflavone von 0,1 bis 4 Gew.-% und Methylricinoleat von 80 bis 90 Gew.-%.

2. Zusammensetzung nach Anspruch 1, wobei die Phytosterole in Mengen im Bereich von 12 bis 17 Gew.-% vorhanden sind, die Isoflavone in Mengen im Bereich von 1 bis 2 Gew.-% vorhanden sind und das Methylricinoleat in Mengen im Bereich von 82 bis 85 Gew.-% vorhanden sind.

3. Zusammensetzung nach den Ansprüchen 1 oder 2, wobei die Phytosterole ein pflanzlicher Phytosterolextrakt sind, der 40 bis 60 Gew.-% Sitosterol, 18 bis 30 Gew.-% Campesterol und 15 bis 28 Gew.-% Stigmasterol umfasst.

4. Zusammensetzung nach einem der Ansprüche 1 bis 3, wobei die Isoflavone ein pflanzlicher Isoflavonextrakt sind, der ungefähr 100 Gew.-% Isoflavone umfasst.

5. Zusammensetzung nach den Ansprüchen 3 oder 4, wobei die pflanzlichen Phytosterol- und Isoflavonextrakte Sojabohnenextrakte sind.

6. Kosmetische Formulierung umfassend eine aktive kosmetische Zusammensetzung nach einem der Ansprüche 1 bis 5 zusammen mit kosmetisch verträglichen Exzipienten.

7. Formulierung nach Anspruch 6 umfassend die aktive kosmetische Zusammensetzung nach einem der Ansprüche 1 bis 5 in Mengen im Bereich von 1 bis 30 Gew.-%, vorzugsweise von 2 bis 20 Gew.-%.

8. Formulierung nach den Ansprüchen 6 oder 7, wobei die Exzipienten ausgewählt sind aus der Gruppe bestehend aus: Lösungsmitteln, Erweichungsmitteln, hygroskopischen Mitteln, Hydratisierungsmitteln, Filmbildnern, Konservierungsmitteln, oberflächenaktiven Stoffen, Viskositätsreglern, Antioxidationsmitteln, Emulgatoren, Duftstoffen, Puffern, Chelatbildnern, Konditionierungsmitteln und Mischungen davon.

9. Formulierung nach einem der Ansprüche 6 bis 8, wobei die Exzipienten ausgewählt sind aus der Gruppe bestehend aus: PPG-15-Stearylether, Isohexadecan, Dimethicon, Ethylhexylethylhexanoat, Cocoglyceriden, Lecithin, Propylenglycol, Glycerin, Hexyldecanol, Betain, Glycerylpolymethacrylat, Acrylaten/C10-30-Alkylacrylat-Kreuzpolymer, Polyacrylamid, Polymethylmethacrylat, Phenoxyethanol, Methylparaben, Butylparaben, Ethylparaben, Propylparaben und Isobutylparaben, Carbomer, Dinatrium-EDTA, Aminomethylpropanol, Citronensäure, Duftstoff, Cetearylalkohol, Cetearylglucosid, Kalium-cetylphosphat, Laureth-7, Wasser, C13-C14-Isoparaffin, Ceramid 3, Tocopherol, Ascorbylpalmitat, Ethylxymeninat und Mischungen davon.

10. Formulierung nach Anspruch 9, wobei die Exzipienten in den in der folgenden Tabelle angegebenen Mengen vorhanden sind:
| **Exzipient** **(Chemischer Name oder INCI-EU-Name)** | **Rolle** | **Menge (Gew.-%)** | **Bevorzugte Menge (Gew.-%)** |
|---|---|---|---|
| WASSER | Lösungsmittel | bis 100 | bis 100 |
| PPG-15-STEARYL-ETHER | Erweichungsmittel | 2-9 | 5-9 |
| ISOHEXADECAN | Erweichungsmittel | 2-9 | 5-9 |
| PROPYLENGLYCOL | Hygroskopisches Mittel | 0,1-6 | 2-5 |
| BETAIN | Hydratisierungsmittel | 0,1-3 | 0,5-2,0 |
| ETHYL-XYMENINAT | Konditionierungsmittel | 0,01-1 | 0,02-0,5 |
| GLYCERYL-POLYMETHACRYLAT | Filmbildner | 0,5-2 | 0,5-1 |
| DIMETHICON | Erweichungsmittel | 0,1-3 | 1-2 |
| PHENOXYETHANOL | Konservierungsmittel | 0,1-0,9 | 0,6-0,8 |
| KALIUM-CETYL-PHOSPHAT | Oberflächenaktiver Stoff | 0,1-2 | 0,1-0,6 |
| CARBOMER | Viskositätsregler | 0,1-2 | 0,5-1,0 |
| DUFTSTOFF | Duftstoff | 0,1-1,0 | 0,1-0,3 |
| AMINOMETHYL-PROPANOL | Puffer | 0,1-1 | 0,1-0,5 |
| ACRYLATE/C10-30-ALKYLACRYLAT-KREUZPOLYMER | Filmbildner | 0,1-3 | 0,1-1 |
| METHYLPARABEN | Konservierungsmittel | 0,05-0,2 | 0,1-0,2 |
| DINATRIUM-EDTA | Chelatbildner | 0,05-0,1 | 0,1 |
| BUTYLPARABEN | Konservierungsmittel | 0,01-0,2 | 0,1-0,2 |
| ETHYLPARABEN | Konservierungsmittel | 0,01-0,2 | 0,01-0,05 |
| PROPYLPARABEN | Konservierungsmittel | 0,01-0,2 | 0,01-0,05 |
| ISOBUTYLPARABEN | Konservierungsmittel | 0,01-0,2 | 0,01-0,05 |
| ETHYLHEXYL-ETHYLHEXANOAT | Erweichungsmittel | 4-12 | 5-9 |
| COCOGLYCERIDE | Erweichungsmittel/ Emulgator | 4-12 | 5-9 |
| GLYCERIN | Denaturierungsmittel/ Hygroskopisches Mittel/ Lösungsmittel | 1-5 | 2-3 |
| HEXYLDECANOL | Hygroskopisches Mittel | 0,5-3 | 0,5-1 |
| POLYACRYLAMID | Antistatikmittel/Bindemittel/ Filmbildner | 0,2-2 | 0,5-1 |
| C13-14-ISOPARAFFIN | Lösungsmittel | 0,01-0,2 | 0,01-0,5 |
| LAURETH-7 | Emulgator/ Oberflächenaktiver Stoff | 0,01-0,2 | 0,01-0,5 |
| LECITHIN | Konditionierungsmittel/ Erweichungsmittel/ Emulgator | 0,01-0,2 | 0,01-0,5 |
| CERAMID 3 | Konditionierungsmittel | 0,01-1,0 | 0,01-0,5 |
| TOCOPHEROL | Antioxidationsmittel | 0,001-1,0 | 0,05-0,5 |
| ASCORBYL-PALMITAT | Antioxidationsmittel | 0,001-1,0 | 0,05-0,5 |
| CITRONENSÄURE | Puffer/Chelatbildner | 0,001-0,1 1 | 0,05-0,1 |
| DIMETHICON, POLYMETHYL-METHACRYLAT, POLYACRYLAMID | Polymere, Filmbildner und Silicone | 0,5-5,0 | 0,5-2 |

11. Kosmetische Formulierung nach einem der Ansprüche 6 bis 10 mit der in der folgenden Tabelle gezeigten Zusammensetzung:
| **Chemischer Name oder INCI-EU-Name** | **Rolle** | **Menge %** |
|---|---|---|
| WASSER | Lösungsmittel | bis 100 |
| PPG-15-STEARYL-ETHER | Erweichungsmittel | 2-9 |
| ISOHEXADECAN | Erweichungsmittel | 2-9 |
| PROPYLENGLYCOL | Hygroskopisches Mittel | 0,1-6 |
| BETAIN | Hydratisierungsmittel | 0,1-3 |
| ETHYL-XYMENINAT | Konditionierungsmittel | 0,01-1 |
| GLYCERYL-POLYMETHACRYLAT | Filmbildner | 0,5-2 |
| DIMETHICON | Erweichungsmittel | 0,1-3 |
| Kosmetische Zusammensetzung umfassend: 13 bis 17 Gew.-% Phytosterole, 1 bis 2 Gew.-% Isoflavone und 80 bis 88 Gew.-% Methylricinoleat | Wirkstoff | 4-10 |
| PHENOXYETHANOL | Konservierungsmittel | 0,1-0,9 |
| KALIUM-CETYLPHOSPHAT | Oberflächenaktiver Stoff | 0,1-2 |
| CARBOMER | Viskositätsregler | 0,1-2 |
| DUFTSTOFF | Duftstoff | nach Bedarf |
| AMINOMETHYLPROPANOL | Puffer | 0,1-1 |
| ACRYLATE/C10-30-ALKYLACRYLAT- KREUZPOLYMER | Filmbildner | 0,1-3 |
| METHYLPARABEN | Konservierungsmittel | 0,05-0,2 |
| DINATRIUM-EDTA | Chelatbildner | 0,05-0,1 |
| BUTYLPARABEN | Konservierungsmittel | 0,01-0,2 |
| ETHYLPARABEN | Konservierungsmittel | 0,01 |
| PROPYLPARABEN | Konservierungsmittel | 0,01 |
| ISOBUTYLPARABEN | Konservierungsmittel | 0,01 |

12. Kosmetische Formulierung nach einem der Ansprüche 6 bis 10 mit der in der folgenden Tabelle gezeigten Zusammensetzung:
| **Chemischer Name oder INCI-EU-Name** | **Rolle** | **Menge %** |
|---|---|---|
| WASSER | Lösungsmittel | bis 100 |
| ETHYLHEXYLETHYLHEXANOAT | Erweichungsmittel | 4-12 |
| COCOGLYCERIDE | Erweichungsmittel/ Emulgator | 4-12 |
| GLYCERIN | Denaturierungsmittel/ Hygroskopisches Mittel/ Lösungsmittel | 1-5 |
| DIMETHICON | Antischaummittel/ Erweichungsmittel | 0,5-5 |
| Kosmetische Zusammensetzung umfassend: 13 bis 17 Gew.-% Phytosterole, 1 bis 2 Gew.-% Isoflavone und 80 bis 88 Gew.-% Methylricinoleat | Wirkstoff | 1-10 |
| HEXYLDECANOL | Hygroskopisches Mittel | 0,5-3 |
| PHENOXYETHANOL | Konservierungsmittel | 0,1-0,9 |
| DUFTSTOFF | Deodorant | nach Bedarf |
| KALIUM-CETYLPHOSPHAT | Oberflächenaktiver Stoff | 0,500 |
| AMINOMETHYLPROPANOL | Puffer | 0,1-2 |
| CARBOMER | Viskositätsregler | 0,1-2 |
| ACRYLATE/C10-30-ALKYLACRYLAT-KREUZPOLYMER | Filmbildner | 0,1-2 |
| POLYACRYLAMID | Antistatikmittel/ Bindemittel/Filmbildner | 0,21-2 |
| METHYLPARABEN | Konservierungsmittel | 0,01-0,2 |
| C13-14-ISOPARAFFIN | Lösungsmittel | 0,01-0,2 |
| DINATRIUM-EDTA | Chelatbildner/ Viskositätsregler | 0,01-0,2 |
| LAURETH-7 | Emulgator/ Oberflächenaktiver Stoff | 0,01-0,2 |
| BUTYLPARABEN | Konservierungsmittel | 0,01-0,2 |
| ETHYLPARABEN | Konservierungsmittel | 0,01-0,2 |
| LECITHIN | Konditionierungsmittel/ Erweichungsmittel/ Emulgator | 0,01-0,2 |
| CERAMID 3 | Konditionierungsmittel | 0,01-0,2 |
| PROPYLPARABEN | Konservierungsmittel | 0,01-0,2 |
| ISOBUTYLPARABEN | Konservierungsmittel | 0,01-0,2 |
| TOCOPHEROL | Antioxidationsmittel | 0,001-0,1 |
| ASCORBYLPALMITAT | Antioxidationsmittel | 0,001-0,1 |
| CITRONENSÄURE | Puffer/Chelatbildner | 0,001-0,1 |

13. Kosmetische Formulierung nach einem der Ansprüche 6 bis 12 mit der in der folgenden Tabelle gezeigten Zusammensetzung:
| **Chemischer Name oder INCI-EU-Name** | **Rolle** | **Menge %** |
|---|---|---|
| WASSER | Lösungsmittel | bis 100 |
| PPG-15-STEARYLETHER | Erweichungsmittel | 7,00 |
| ISOHEXADECAN | Erweichungsmittel | 6,000 |
| CETEARYLALKOHOL | Emulgator | 4,000 |
| GLYCERIN | Hygroskopisches Mittel | 2,000 |
| CETEARYLGLUCOSID | Emulgator | 1,000 |
| DIMETHICON | Antischaummittel | 1,000 |
| PHENOXYETHANOL | Konservierungsmittel | 0,700 |
| Kosmetische Zusammensetzung umfassend: 15 % Phytosterole, 1 % Isoflavone und 84 % Me-thylricinoleat | Wirkstoff | 6,000 |
| KALIUM-CETYLPHOSPHAT | Oberflächenaktiver Stoff | 0,500 |
| DUFTSTOFF | Duftstoff | 0,500 |
| ETHYLHEXYLGLYCERIN | Hygroskopisches Mittel | 0,500 |
| POLYACRYLAMID | Texturierender Faktor | 0,200 |
| METHYLPARABEN | Konservierungsmittel | 0,160 |
| C13-14-ISOPARAFFIN | Lösungsmittel | 0,120 |
| DINATRIUM-EDTA | Chelatbildner | 0,1000 |
| LAURETH-7 | Emulgator/ Oberflächenaktiver Stoff | 0,040 |
| BUTYLPARABEN | Konservierungsmittel | 0,040 |
| ETHYLPARABEN | Konservierungsmittel | 0,040 |
| PROPYLPARABEN | Konservierungsmittel | 0,020 |
| ISOBUTYLPARABEN | Konservierungsmittel | 0,020 |

## Revendications

1. Composition cosmétique comprenant des phytostérols dans des quantités comprises entre 1 et 20 % en poids, des isoflavones entre 0,1 et 4 % en poids et du ricinoléate de méthyle entre 80 et 90 % en poids.

2. Composition selon la revendication 1, dans laquelle lesdits phytostérols sont présents dans des quantités comprises entre 12 et 17 % en poids, lesdites isoflavones sont présentes dans des quantités comprises entre 1 et 2 % en poids, et ledit ricinoléate de méthyle est présent dans des quantités comprises entre 82 et 85 % en poids.

3. Composition selon la revendication 1 ou 2, dans laquelle lesdits phytostérols sont un extrait de phytostérol végétal comprenant de 40 à 60 % en poids de sitostérol, de 18 à 30 % en poids de campestérol et de 15 à 28 % en poids de stigmastérol.

4. Composition selon l'une quelconque des revendications 1 à 3, dans laquelle lesdites isoflavones sont un extrait d'isoflavone végétal comprenant approximativement 100 % en poids d'isoflavones.

5. Composition selon la revendication 3 ou 4, dans laquelle lesdits extraits de phytostérol et d'isoflavone végétaux sont des extraits de soja.

6. Formulation cosmétique comprenant une composition cosmétique active selon l'une quelconque des revendications 1 à 5 avec des excipients cosmétiquement acceptables.

7. Formulation selon la revendication 6 comprenant la composition cosmétique active selon l'une quelconque des revendications 1 à 5 dans des quantités comprises entre 1 et 30 % en poids, de préférence de 2 à 20 % en poids.

8. Formulation selon la revendication 6 ou 7, dans laquelle lesdits excipients sont choisis dans le groupe constitué : de solvants, d'émollients, d'agents hygroscopiques, d'agents hydratants, d'agents filmogènes, de conservateurs, de tensioactifs, d'agents de régulation de la viscosité, d'antioxydants, d'émulsionnants, de parfums, de tampons, d'agents chélatants, de conditionneurs et de mélanges de ceux-ci.

9. Formulation selon l'une quelconque des revendications 6 à 8, dans laquelle lesdits excipients sont choisis dans le groupe constitué : de PPG-15 stéaryléther, d'isohexadécane, de diméthicone, d'éthylhexanoate d'éthylhexyle, de cocoglycérides, de lécithine, de propylèneglycol, de glycérine, d'hexyldécanol, de bétaïne, de poly(méthacrylate de glycéryle), d'un copolymère d'acrylates/acrylate d'alkyle en C10-30, de polyacrylamide, de poly(méthacrylate de méthyle), de phénoxyéthanol, de méthylparabène, de butylparabène, d'éthylparabène, de propylparabène et d'isobutylparabène, de carbomère, de disodium EDTA, d'aminométhyl-propanol, d'acide citrique, de parfum, d'alcool cétéarylique, de cétéaryl-glucoside, de cétylphosphate de potassium, de laureth-7, d'eau, d'isoparaffine en C13-C14, de céramide 3, de tocophérol, de palmitate d'ascorbyle, de xyméninate d'éthyle et de mélanges de ceux-ci.

10. Formulation selon la revendication 9, dans laquelle lesdits excipients sont présents dans les quantités citées dans le tableau suivant :
| Excipient (nom chimique ou nom INCI-EU) | Rôle | Quantité (% en poids) | Quantité préférée (% en poids) |
|---|---|---|---|
| EAU | Solvant | jusqu'à 100 | jusqu'à 100 |
| PPG-15 STEARYLETHER | Emollient | 2-9 | 5-9 |
| ISOHEXADECANE | Emollient | 2-9 | 5-9 |
| PROPYLENEGLYCOL | Agent hygroscopique | 0,1-6 | 2-5 |
| BETAINE | Agent hydratant | 0,1-3 | 0,5-2,0 |
| XYMENINATE D'ETHYLE | Conditionneur | 0,01-1 | 0,02-0,5 |
| POLY(METHACRYLATE DE GLYCERYLE) | Agent filmogène | 0,5-2 | 0,5-1 |
| DIMETHICONE | Emollient | 0,1-3 | 1-2 |
| PHENOXYETHANOL | Conservateur | 0,1-0,9 | 0,6-0,8 |
| CETYLPHOSPHATE DE POTASSIUM | Tensioactif | 0,1-2 | 0,1-0,8 |
| CARBOMERE | Agent de régulation de la viscosité | 0,1-2 | 0,5-1,0 |
| PARFUM | Parfum | 0,1-1,0 | 0,1-0,3 |
| AMINOMETHYLPROPANOL | Tampon | 0,1-1 | 0,1-0,5 |
| COPOLYMERE D'ACRYLATES/ACRYLATE D'ALKYLE EN C10-30 | Agent filmogène | 0,1-3 | 0,1-1 |
| METHYLPARABENE | Conservateur | 0,05-0,2 | 0,1-0,2 |
| DISODIUM EDTA | Agent chélatant | 0,05-0,1 | 0,1 |
| BUTYLPARABENE | Conservateur | 0,01-0,2 | 0,1-0,2 |
| ETHYLPARABENE | Conservateur | 0,01-0,2 | 0,01-0,05 |
| PROPYLPARABENE | Conservateur | 0,01-0,2 | 0,01-0,05 |
| ISOBUTYLPARABENE | Conservateur | 0,01-0,2 | 0,01-0,05 |
| ETHYLHEXANOATE D'ETHYLHEXYLE | Emollient | 4-12 | 5-9 |
| COCOGLYCERIDES | Emollient/ émulsionnant | 4-12 | 5-9 |
| GLYCERINE | Dénaturant/ agent hygroscopique/ solvant | 1-5 | 2-3 |
| HEXYLDECANOL | Agent hygroscopique | 0,5-3 | 0,5-1 |
| POLYACRYLAMIDE | Agent antistatique/ agent liant/agent filmogène | 0,2-2 | 0,5-1 |
| ISOPARAFFINE EN C13-C14 | Solvant | 0,01-0,2 | 0,01-0,5 |
| LAURETH-7 | Emulsionnant/ tensioactif | 0,01-0,2 | 0,01-0,5 |
| LECITHINE | Conditionneur/émollient/ émulsionnant | 0,01-0,2 | 0,01-0,5 |
| CERAMIDE 3 | Conditionneur | 0,01-1,0 | 0,01-0,5 |
| TOCOPHEROL | Antioxydant | 0,001-1,0 | 0,05-0,5 |
| PALMITATE D'ASCORBYLE | Antioxydant | 0,001-1,0 | 0,05-0,5 |
| ACIDE CITRIQUE | Tampon/agent chélatant | 0,001-0,1 | 0,05-0,1 |
| DIMETHICONE, POLY(METHACRYLATE DE METHYLE) POLYACRYLAMIDE | Polymères, agent filmogène et silicones | 0,5-5,0 | 0,5-2 |

11. Formulation cosmétique selon l'une quelconque des revendications 6 à 10 présentant la composition représentée dans le tableau suivant :
| Nom chimique ou nom INCI-EU | Rôle | Quantité en % |
|---|---|---|
| EAU | Solvant | Jusqu'à 100 |
| PPG-15 STEARYLETHER | Emollient | 2-9 |
| ISOHEXADECANE | Emollient | 2-9 |
| PROPYLENEGLYCOL | Agent hygroscopique | 0,1-6 |
| BETAINE | Agent hydratant | 0,1-3 |
| XYMENINATE D'ETHYLE | Conditionneur | 0,01-1 |
| POLY(METHACRYLATE DE GLYCERYLE) | Agent filmogène | 0,5-2 |
| DIMETHICONE | Emollient | 0,1-3 |
| Composition cosmétique comprenant : de 13 à 17 % en poids de phytostérols, de 1 à 2 % en poids d'isoflavones et de 80 à 88 % en poids de ricinoléate de méthyle | Principe actif | 4-10 |
| PHENOXYETHANOL | Conservateur | 0,1-0,9 |
| CETYLPHOSPHATE DE POTASSIUM | Tensioactif | 0,1-2 |
| CARBOMERE | Agent de régulation de la viscosité | 0,1-2 |
| PARFUM | Parfum | si nécessaire |
| AMINOMETHYLPROPANOL | Tampon | 0,1-1 |
| COPOLYMERE D'ACRYLATES/ACRYLATE D'ALKYLE EN C10-30 | Agent filmogène | 0,1-3 |
| METHYLPARABENE | Conservateur | 0,05-0,2 |
| DISODIUM EDTA | Agent chélatant | 0,05-0,1 |
| BUTYLPARABENE | Conservateur | 0,01-0,2 |
| ETHYLPARABENE | Conservateur | 0,01 |
| PROPYLPARABENE | Conservateur | 0,01 |
| ISOBUTYLPARABENE | Conservateur | 0,01 |

12. Formulation cosmétique selon l'une quelconque des revendications 6 à 10 présentant la composition représentée dans le tableau suivant :
| Nom chimique ou nom INCI-EU | Rôle | Quantité en % |
|---|---|---|
| EAU | Solvant | Jusqu'à 100 |
| ETHYLHEXANOATE D'ETHYLHEXYLE | Emollient | 4-12 |
| COCOGLYCERIDES | Emollient/émulsionnant | 4-12 |
| GLYCERINE | Dénaturant/agent hygroscopique/solvant | 1-5 |
| DIMETHICONE | Agent antimoussant/émollient | 0,5-5 |
| Composition cosmétique comprenant : de 13 à 17 % en poids de phytostérols, de 1 à 2 % en poids d'isoflavones et de 80 à 88 % en poids de ricinoléate de méthyle | Principe actif | 1-10 |
| HEXYLDECANOL | Agent hygroscopique | 0,5-3 |
| PHENOXYETHANOL | Conservateur | 0,1-0,9 |
| PARFUM | Déodorant | si nécessaire |
| CETYLPHOSPHATE DE POTASSIUM | Tensioactif | 0,500 |
| AMINOMETHYLPROPANOL | Tampon | 0,1-2 |
| CARBOMERE | Agent de régulation de la viscosité | 0,1-2 |
| COPOLYMERE D'ACRYLATES/ ACRYLATE D'ALKYLE EN C10-30 | Agent filmogène | 0,1-2 |
| POLYACRYLAMIDE | Agent antistatique/agent liant/ agent filmogène | 0,21-2 |
| METHYLPARABENE | Conservateur | 0,01-0,2 |
| ISOPARAFFINE EN C13-14 | Solvant | 0,01-0,2 |
| DISODIUM EDTA | Agent chélatant/agent de régulation de la viscosité | 0,01-0,2 |
| LAURETH-7 | Emulsionnant/tensioactif | 0,01-0,2 |
| BUTYLPARABENE | Conservateur | 0,01-0,2 |
| ETHYLPARABENE | Conservateur | 0,01-0,2 |
| LECITHINE | Conditionneur/émollient/ Emulsionnant | 0,01-0,2 |
| CERAMIDE 3 | Conditionneur | 0,01-0,2 |
| PROPYLPARABENE | Conservateur | 0,01-0,2 |
| ISOBUTYLPARABENE | Conservateur | 0,01-0,2 |
| TOCOPHEROL | Antioxydant | 0,001-0,1 |
| PALMITATE D'ASCORBYLE | Antioxydant | 0,001-0,1 |
| ACIDE CITRIQUE | Tampon/agent chélatant | 0,001-0,1 |

13. Formulation cosmétique selon l'une quelconque des revendications 6 à 12 présentant la composition représentée dans le tableau suivant :
| Nom chimique ou nom INCI-EU | Rôle | Quantité en % |
|---|---|---|
| EAU | Solvant | Jusqu'à 100 |
| PPG-15 STEARYLETHER | Emollient | 7,00 |
| ISOHEXADECANE | Emollient | 6,000 |
| ALCOOL CETEARYLIQUE | Emulsionnant | 4,000 |
| GLYCERINE | Agent hygroscopique | 2,000 |
| CETEARYLGLUCOSIDE | Emulsionnant | 1,000 |
| DIMETHICONE | Agent anti-mousse | 1,000 |
| PHENOXYETHANOL | Conservateur | 0,700 |
| Composition cosmétique comprenant : 15 % de phytostérols, 1 % d'isoflavones et 84 % de ricinoléate de méthyle | Principe actif | 6,000 |
| CETYLPHOSPHATE DE POTASSIUM | Tensioactif | 0,500 |
| PARFUM | Parfum | 0,500 |
| ETHYLHEXYLGLYCERINE | Agent hygroscopique | 0,500 |
| POLYACRYLAMIDE | Facteur de texture | 0,200 |
| METHYLPARABENE | Conservateur | 0,160 |
| ISOPARAFFINE EN C13-14 | Solvant | 0,120 |
| DISODIUM EDTA | Agent chélatant | 0,1000 |
| LAURETH-7 | Emulsionnant/ tensioactif | 0,040 |
| BUTYLPARABENE | Conservateur | 0,040 |
| ETHYLPARABENE | Conservateur | 0,040 |
| PROPYLPARABENE | Conservateur | 0,020 |
| ISOBUTYLPARABENE | Conservateur | 0,020 |
